(19) **Europäisches Patentamt**
**European Patent Office**
**Office européen des brevets**

(11) **EP 4 397 327 A1**

(12) **EUROPEAN PATENT APPLICATION**
published in accordance with Art. 153(4) EPC

(43) Date of publication:
**10.07.2024 Bulletin 2024/28**

(21) Application number: **22864530.5**

(22) Date of filing: **30.08.2022**

(51) International Patent Classification (IPC):
*A61L 27/02* (2006.01)    *A61C 8/00* (2006.01)
*A61L 27/04* (2006.01)    *A61L 27/06* (2006.01)
*A61L 27/42* (2006.01)    *A61L 27/54* (2006.01)

(52) Cooperative Patent Classification (CPC):
**A61C 8/00; A61L 27/02; A61L 27/04; A61L 27/06;**
**A61L 27/42; A61L 27/54**

(86) International application number:
**PCT/JP2022/032507**

(87) International publication number:
**WO 2023/032948 (09.03.2023 Gazette 2023/10)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **31.08.2021 JP 2021140957**

(71) Applicant: **Maruemu Works Co., Ltd.**
**Osaka-shi, Osaka 5420086 (JP)**

(72) Inventors:
• **NARITA, Kengo**
**Daitou-shi, Osaka 574-0015 (JP)**
• **YAMANAKA, Shigeru**
**Daitou-shi, Osaka 574-0015 (JP)**

(74) Representative: **Meissner Bolte Partnerschaft**
**mbB**
**Widenmayerstraße 47**
**80538 München (DE)**

(54) **BIOCOMPATIBLE FILM AND BIOCOMPATIBLE MATERIAL HAVING SAID FILM**

(57)    The present invention provides a biocompatible film having biological safety and a stable effect. The present invention provides a biocompatible film containing magnesium and optionally containing calcium, in which the weight of calcium is 0 to 40% by weight relative to the total weight, i.e., 100% by weight, of magnesium and calcium, the biocompatible film having such antibacterial properties that the antibacterial activity value is 2.0 or more.

Fig. 3

EP 4 397 327 A1

**Description**

Technical Field

The present invention relates to a biocompatible film comprising magnesium and optionally calcium, wherein the biocompatible film has an antibacterial property.
Further, the present invention relates to a biocompatible material having the biocompatible film.

Background Art

The treatment of teeth by implants has attracted attention as a type of treatment method for tooth loss for young people to elderly people.
On the other hand, peri-implantitis caused by bacterial adhesion is a clinical problem in implant treatment. Implants made of metal or ceramics do not have an antibacterial property at all, and thus, in a case where bacteria adhere to them during or after surgery, inflammation occurs around them, causing rapid bone resorption and often causing the implants to come off. Therefore, there is a demand for a technique for imparting an antibacterial property to implants.
For example, Patent Document 1 discloses a technique for forming an Ag-doped anodized film to impart an antibacterial property. However, since Ag is highly toxic, only a very small amount of Ag can be added, and thus, uniform doping is difficult.
Patent Document 2 discloses an antibacterial technique using photocatalysis. However, photocatalysts do not have antibacterial property in the absence of light, and thus, they cannot exhibit their antibacterial properties sufficiently, when they are implanted in the body.
In Patent Document 3, the substrate surface is coated with hydroxyapatite (HAP), and magnesium, strontium, gallium, zinc, copper, silver, europium, and terbium are contained therein as metals that improve an antibacterial property. In the document, a material having an antibacterial property is produced by immersing a substrate having a surface coated with HAP in an aqueous solution of salts of these metals, impregnating the substrate, and drying the substrate. However, it is very difficult to control the content of the metals, and it is difficult to evenly distribute the metals. Therefore, it has been difficult to stably exhibit the antibacterial property.

Prior Art Document

Patent Document

Patent Document 1: JP Patent No. 6130301.
Patent Document 2: JP-A No. 2008-161424.
Patent Document 3: JP-A No. 2021-7741.

Summary of Invention

Problems to be solved by the Invention

Thus, the prior art cited in Patent Documents 1 to 3 proposes biocompatible materials that impart an antibacterial property. However, since the proposed techniques use highly toxic elements exhibiting the antibacterial property, or since the amount doped of the elements, each of which exhibits the antibacterial property, into the film is very small, there is a problem that the biosafety is low and/or that a stable effect cannot be expected.
Accordingly, an object of the present invention is to provide a biocompatible film that is biosafe and has a stable effect.
Specifically, an object of the present invention is to provide a biocompatible film that is mainly composed of magnesium and calcium that exhibit low toxicity and an antibacterial property, and that can stably exhibit high antibacterial property by dissolving itself.
Other than or in addition to the above object, another object of the present invention is to provide a biocompatible material, such as a dental material, specifically an implant material, comprising the biocompatible film.

Means for Solving Problems

The present inventors have found the following inventions:

<1> A biocompatible film comprising magnesium and optionally calcium, wherein calcium has 0 to 40 % by weight, preferably 0.8 to 35 % by weight, more preferably 5 to 35 % by weight, most preferably 15 to 35 % by weight, where a total weight of magnesium and calcium is 100 % by weight, and the film has an antibacterial property having an antibacterial activity value of 2.0 or more, preferably 2.5 or more, more preferably 3.0 or more, most preferably 3.0 to 5.0.

<2> In the above item <1>, the film may have the antibacterial property in which an average of a viable bacteria count of Staphylococcus aureus at 24 hours after inoculating 0.16 $\mu$l/mm$^2$ of a test bacterial solution having a viable bacteria count of Staphylococcus aureus of $2.5 \times 10^5$ to $10 \times 10^5$ cells/ml onto the film is 75 % or less, preferably 50 % or less, more preferably 30 % or less of the viable bacteria count at the time of inoculation.

<3> A biocompatible film comprising magnesium and optionally calcium, wherein calcium has 0 to 40 % by weight, preferably 0.8 to 35 % by weight, more preferably 5 to 35 % by weight, most preferably 15 to 35 % by weight, where a total weight of magnesium and calcium is 100 % by weight, and the film has an antibacterial property in which an average of a viable bacteria count of Staphylococcus aureus at 24 hours after inoculating 0.16 $\mu$l/mm$^2$ of a test bacterial solution having a viable bacteria count of Staphylococcus aureus of $2.5 \times 10^5$ to $10 \times 10^5$ cells/ml onto the film is 75 % or less, preferably 50 % or less, more preferably 30 % or less of the viable bacteria count at the time of inoculation.

<4> In any one of the above items <1> to <3>, the biocompatible film may have a dissolution property such that the biocompatible film dissolves in a Hank's balanced salt solution.

<5> In any one of the above items <1> to <4>, the biocompatible film may be free from $Mg_2Ca$.

<6> In any one of the above items <1> to <5>, the biocompatible film may have an amorphous portion.

<7> A biocompatible material comprising the biocompatible film in any one of the above items <1> to <6>; and a biocompatible substrate.

<8> In the above item <7>, the biocompatible substrate may be at least one selected from the group consisting of pure titanium, a cobalt-chromium alloy, stainless steel, titanium alloys, zirconia, alumina, calcium phosphate and magnesia.

<9> In the above item <7> or <8>, a surface roughness Ra of the biocompatible substrate may be 50 um or less, preferably 40 um or less, more preferably 30 um or less.

<10> In any one of the above items <7> to <9>, a shape of the biocompatible material may be one selected from the group consisting of a cylindrical shape, a truncated cone shape, a conical shape, a shape having a screw-shaped threaded portion in a part of the shape, a rectangular parallelepiped and a cube, a block shape having a partially inclined surface, and a wedge shape.

<11> In any one of the above items <7> to <10>, the biocompatible material may be one selected from the group consisting of an artificial bone material, an intraosseous fixture material, a dental implant material, an orthodontic anchor screw material, a medullary nail material, and an interbody fixation material.

Effects of the Invention

The present invention can provide a biocompatible film that is biosafe and has a stable effect.

Specifically, the present invention can provide a biocompatible film that is mainly composed of magnesium and calcium that exhibit low toxicity and an antibacterial property, and that can stably exhibit high antibacterial property by dissolving itself.

Other than or in addition to the above effects, the present invention can provide a biocompatible material, such as a dental material, specifically an implant material, comprising the biocompatible film.

Brief Description of Drawings

Fig. 1 is a graph showing X-ray diffraction profiles for an Mg film, an Mg10%Ca film, an Mg20%Ca film, and an Mg30%Ca film, each of which were obtained by subjecting ion irradiation to the glass substrate B, followed by sputtering using only magnesium (Mg), 10 % by weight (Mg10%Ca), 20 % by weight (Mg20%Ca) and 30 % by weight (Mg30%Ca) of an amount of Ca as a target.

Fig. 2 is a graph showing X-ray diffraction profiles for an Mg film, an Mg20%Ca film, and an Mg30%Ca film, each of which were obtained by sputtering using plate zirconia A-F and rough plate zirconia A-R.

Fig. 3 is a diagram showing the pH measurement results for evaluating the dissolution rate in HBSS(-) immersion for smooth zirconia A-F2 with Mg film, Mg20%Ca film, and Mg30%Ca film.

Description of Embodiments

The invention described in the present application (hereinafter, may be abbreviated as the "present invention") is described hereinafter.

The present application provides a biocompatible film comprising magnesium and optionally calcium, wherein calcium has 0 to 40 % by weight, preferably 0.8 to 35 % by weight, more preferably 5 to 35 % by weight, most preferably 15 to 35 % by weight, where a total weight of magnesium and calcium is 100 % by weight, and the film has an antibacterial property.

In addition, the present application provides a biocompatible material comprising the biocompatible film; and a biocompatible substrate.

< A biocompatible film>

The biocompatible film according to the present application comprises magnesium and optionally calcium, and calcium may have 0 to 40 % by weight, preferably 0.8 to 35 % by weight, more preferably 5 to 35 % by weight, further preferably 15 to 35 % by weight, most preferably 25 to 35 % by weight, where a total weight of magnesium and calcium is 100 % by weight. The higher the calcium content, the larger the region of the amorphous structure. In a case where the calcium content is 10% by weight, a sharp peak derived from crystals is observed in the amorphous structure. However, in a case where the calcium content is 20% by weight, a broad halo pattern derived from the amorphous structure is observed. A low-intensity peak that seems to be derived from microcrystals is observed in it. In a case of 25% by weight or more, almost no crystal-derived peaks are observed. In a case of 30% by weight, only a broad halo pattern derived from an amorphous structure is observed. When the biocompatible film of the present application comprises calcium, a large portion thereof has a substantially amorphous structure. As the portions of the amorphous structure increase, crystals such as $Mg_2Ca$ are difficult to form, and the biocompatible film dissolves uniformly in body fluids or simulated body fluids, exhibiting a stable antibacterial property.

When the film is manufactured by sputtering, the content of calcium may be within 35 % by weight in order easily to manufacture the target.

The biocompatible film according to the present application can exhibit desired biosafety.

The biocompatible film according to the present application may consist essentially of magnesium and optionally calcium. Preferably The biocompatible film may consist of magnesium and optionally calcium. Furthermore, the phrase "consisting of X" means that it is composed only of the components described in "X". Further, the phrase "consisting essentially of X" means that component(s) other than those described in "X" may include, but that the component(s) other than those described in "X" is comprised such that the component(s) does(do) not change the characteristics derived from the substance composed only of those described in "X".

Further, the biocompatible film may be free from $Mg_2Ca$. The term "be free from $Mg_2Ca$" means that a peak based on $Mg_2Ca$ is not observed in X-ray diffraction analysis, and preferably that a peak based on $Mg_2Ca$ is not observed at a diffraction angle at which diffraction peaks generated from crystals of the substrate and $Mg_2Ca$ do not overlap each other (the respective diffraction peaks are separated by 1° or more in X-ray analysis by using a cobalt (Co) tube lamp), for example, a peak is not observed in a range of 36 to 37° in X-ray analysis using a Co tube lamp.

More, the biocompatible film may have an amorphous portion. The term "amorphous" means that no sharp peak is observed in X-ray diffraction analysis.

The biocompatible film according to the present application comprises magnesium and optionally calcium. The components including magnesium and calcium in the film may be evenly distributed. The biocompatible film can stably exhibit an antibacterial property because of even distribution.

<Antibacterial property>

The biocompatible film according to the present application has an antibacterial property.

The antibacterial property may have an antibacterial activity value of 2.0 or more, preferably 2.5 or more, more preferably 3.0 or more, most preferably 3.0 to 5.0.

The antibacterial activity value can be determined according to JIS Z2801 film method.

For example, Staphylococcus aureus may be used for measuring the antibacterial activity value, the number of viable bacteria in the test bacterial solution may be $2.5 \times 10^5$ to $10 \times 10^5$ cells/ml, and the inoculum amount may be, for example, 0.016 mL. A control Sample C which is a non-antimicrobial PE film, a sample A which is a substrate B coated with a biocompatible film according to the present invention, and a sample B which has no coating (i.e., a substrate B) are prepared. The average logarithm of the viable count immediately after inoculation of control sample C (PE film) ($U_0$), the average logarithm of the viable count at 24 hours after inoculation for sample A ($A_t$), and the average logarithm value (Ut) of the viable count of the sample B (substrate B) at 24 hours after inoculation are determined. The antibacterial activity value R can be obtained based on the following formula (1). In addition, in the determination of the viable cell count, if the number of colonies is 1 or less, it is regarded as 1, and the viable cell count per unit area ($cm^2$) of the PE film is calculated.

$$R = (U_t - U_0) - (A_t - U_0) = U_t - A_t \qquad (1)$$

In addition, as an antibacterial property, the average number of viable bacteria of Staphylococcus aureus at 24 hours after inoculating 0.16 $\mu$l/mm$^2$ of a test bacterial solution having a viable count of Staphylococcus aureus of $2.5 \times 10^5$ to $10 \times 10^5$ cells/ml may be 75% or less, preferably 50% or less, more preferably 30% or less of the viable bacteria count at the time of inoculation.

Furthermore, the antibacterial property may have the specific range of the antibacterial activity value and may have the specific range of the average number of viable bacteria of Staphylococcus aureus at 24 hours after inoculating 0.16 $\mu$l/mm$^2$ of a test bacterial solution having a viable count of Staphylococcus aureus of $2.5 \times 10^5$ to $10 \times 10^5$ cells/ml.

The biocompatible film having the above-described specific ranges of the antibacterial property can exhibit stable antibacterial property.

The biocompatible film according to the present application may have the property of dissolving in a body fluid such as blood, lymph, bone marrow fluid, tissue fluid, and the like, and thus, when implanted in the body, the biocompatible film can exhibit the antibacterial property. Further, the biocompatible film according to the present application may have the property of dissolving in a simulated body fluid as well as in the body fluid. The examples of the body fluid may include phosphate buffered saline (PBS), Hank's balanced salt solution (HBSS), SBF solution, plasma liquid, cell culture medium, Eagle (MEM) solution, DMEM solution, serum medium and the like.

<Biocompatible material>

The present application provides a biocompatible material comprising the above-described biocompatible film; and a biocompatible substrate.

<<Biocompatible substrate>>

The biocompatible substrate is not particularly limited as long as it has the above-mentioned "biocompatibility". Examples may include, but are not limited to, metals such as pure titanium, cobalt-chromium alloys, stainless steel, titanium alloys and the like, and ceramics such as zirconia, alumina, calcium phosphate, magnesia and the like. The biocompatible substrate may be preferably pure titanium, titanium alloys and zirconia, more preferably ceramics, and further preferably zirconia. In the present invention, even if the biocompatible substrate is nonconductive ceramics, when the metal film and body fluid or simulated body fluid come into contact with each other, the metal film dissolves and the surface of the biocompatible substrate is exposed, resulting in hydrophilicity.

The biocompatible substrate may have a surface roughness (arithmetic mean roughness) Ra of 50 um or less, preferably 40 um or less, more preferably 30 um or less.

Surface roughness Ra can be determined by a conventionally known method, for example, according to JIS B 0601: 2013. In the standard, a reference length and an evaluation length are defined according to Ra value categories. For example, for 0.1 < Ra(um) ≤ 2, the reference length and evaluation length are 0.8 mm and 2.0 mm, respectively. In a case where the reference length cannot be measured continuously due to interference between the stylus and the object, the reference length in the category lower than the Ra value of the object is used, and the total number of times where the evaluation length is exceeded is measured, and then the average value can be measured as Ra.

The biocompatible substrate may have a surface having a porous structure, grid grooves, or fine dimples.

The biocompatible material according to the present application comprises the above-described biocompatible substrate; and the above-described biocompatible film. However, the biocompatible material according to the present application may have other layers.

For example, one or more layers for hydrophilization may be provided between the biocompatible substrate and the biocompatible film. Examples of the layer may include, but are not limited to, an anodizing layer, a hydrothermal treatment layer, and the like for titanium or a titanium alloy. More, one or more layers may be provided on the upper portion of the above-mentioned film, that is, on the side opposite to the substrate.

The shape of the biocompatible material according to the present invention is not particularly limited, but for example, the biocompatible material may be one selected from the group consisting of a columnar shape, a cylindrical shape, a truncated cone shape, a conical shape, a shape having a screw-shaped threaded portion in a part of the shape, a rectangular parallelepiped and a cube, a block shape such as a rectangular parallelepiped and a cube having a partially inclined surface, and a wedge shape.

The application field of the biocompatible material according to the present invention is not particularly limited, but for example, the biocompatible material may be one selected from the group consisting of an artificial bone material, an intraosseous fixture material, a dental implant material, an orthodontic anchor screw material, a medullary nail material, and an interbody fixation material. Examples thereof may include, but are not limited to, an artificial bone, a pin, wire, a bolt, a screw, a washer, a medullary nail, and a vertebral body spacer.

<Method of producing the biocompatible film and the biocompatible material>

The above-described biocompatible film and the above-described biocompatible material can be produced by the following method. However, the method is not limited as long as the film and the material can be produced so as to have the above-described properties.

A method for producing the biocompatible material is described hereinafter. Since the method also comprises a method for producing a biocompatible film, it also serves as an explanation of the method for producing a biocompatible film.

A method for producing the biocompatible material comprises the step of:

(A) preparing a biocompatible substrate; and
(B) forming a biocompatible film on the surface of the biocompatible substrate.

The step (A) is a step of preparing a biocompatible substrate. The above-described "biocompatible substrate" may be commercially purchased, or the commercially purchased substrate may be formed into a desired shape. The method may comprise a step of grinding and/or polishing the surface of the purchased product or the obtained shape. Furthermore, as the grinding process and the polishing process, conventional processes may be used.

The step (B) is a step of forming a biocompatible film on the surface of the biocompatible substrate.

The step (B) may comprise the steps of:

(B1) preparing a sputtering target comprised of the biocompatible film precursor; and
(B2) sputtering by using the sputtering target, to form the biocompatible film on the biocompatible substrate obtained in the step (A).

In particular, the step (B1) may be (B1a) preparing the sputtering target comprised of magnesium and optional calcium, and

the step (B2) may be (B2a) sputtering by using the sputtering target and setting a temperature of the biocompatible substrate obtained in the step (A) at 130°C or lower, preferably 90°C or lower, more preferably 60°C or lower, to form the biocompatible film comprised of magnesium and optional calcium on the biocompatible substrate, wherein the biocompatible film comprises 0 to 40 % by weight of calcium where a total weight of magnesium and calcium is 100 % by weight.

A magnetron sputtering device may be used as the sputtering device.

In the magnetron sputtering device, a strong magnet (magnetron) is disposed behind a target, and sputtered particles (metal particles) generated by causing argon ions to collide with the target can be efficiently deposited on a substrate using a magnetic field. Upon sputtering, the sputtering voltage, the bias of the substrate, the pressure in the device, and the temperature of a substrate material can be adjusted, to form a film at a constant film formation rate.

The metal film based on magnesium has a linear expansion coefficient three times larger or more than that of zirconium, titanium, or a titanium alloy as the substrate. Thus, when the sputtering temperature is high, the temperature difference from around room temperature at which the implant is used and the sputtering temperature becomes large, and thus, tensile stress (thermal stress) is generated at the interface on the film side, and the film is easily peeled off. Therefore, in order to prevent the film from being peeled off after the film formation and to prevent harmful stress from remaining at the time of implanting the implant, the temperature of the substrate in sputtering is controlled to a certain temperature or less, that is, 130°C or less, preferably 90°C or less, more preferably 60°C or less, so that a film having high adhesiveness can be formed.

The above temperature can be estimated by calculating thermal stress as follows: When the temperature is lowered from the sputtering temperature to room temperature, the stress (thermal stress) at the interface generated can be approximately expressed by Equation 2, wherein $\Delta T$: a temperature difference between a substrate temperature during sputtering Td and room temperature Tr, $\alpha_1$: an average linear expansion coefficient of the substrate between the temperatures Tr and Td, $\alpha_2$: an average linear expansion coefficient of the coating film between the temperatures Tr

and Td, $E_1$: an average elastic modulus of the substrate between the temperatures Tr and Td, and $E_2$: an average elastic modulus of the film between the temperatures Tr and Td.

$$\sigma = \frac{E_1 E_2 (\alpha_1 - \alpha_2) \Delta T}{E_1 (1 - \alpha_2 \Delta T) + E_2 (1 - \alpha_1 \Delta T)} \qquad \text{(Equation 1)}$$

For example, assuming that the substrate is zirconia, and the coating film is pure magnesium, calculation is performed by applying the linear expansion coefficients as $8 \times 10^{-6}$ and $25 \times 10^{-6}$ and the elastic moduli as 210 GPa and 40 GPa, respectively.

Since the yield strength of pure magnesium is generally said to be about 90 to 100 MPa, the shear yield strength is about 50 MPa. In order to suppress the thermal stress to at least this value or less, the temperature difference may be 100 degrees or less. For example, when the operating temperature is 36°C as a body temperature, the film formation temperature may be 130°C or lower, and is preferably 90°C or lower, which is twice the safety factor or 60°C or lower in consideration of about three times the safety factor.

By obtaining the biocompatible film of the present application by sputtering, the components including magnesium and calcium in the film can be distributed almost uniformly as described above. Since it is uniformly distributed, the antibacterial property can be exhibited stably. By using the target in the step (B1a), the components in the film can be distributed more uniformly. The uniformity of the components in the film obtained by sputtering can be confirmed by measuring the elemental distribution, which is not unevenly distributed, with EDX.

The method for producing the biocompatible material may comprise a step(s) other than the above-described steps (A) and (B).

For example, the method may comprise the step of (C) hydrophilizing the surface of the biocompatible substrate after the step (A) and before the step (B). The step may be carried out by at least one selected from the group consisting of acid treatment, blasting treatment, anodizing, hydrothermal treatment, ultraviolet irradiation, plasma irradiation, laser irradiation, radiation irradiation, and ion irradiation.

The method may further comprise the step of (D) ion cleaning the surface of the biocompatible substrate after the step (A) and before the step (B). Furthermore, in a case of comprising the above-described step (C), the step (D) may be carried out after the step (C).

The step of ion cleaning is a step in which impurities on the surface are removed at the atomic level by bombarding the surface of the substrate with argon ions or the like while appropriately adjusting the bias in a vacuum, specifically in a vacuum chamber. By performing the process appropriately, the adhesion of the protected film can be stabilized and the surface of the substrate can be activated.

The producing method may comprise a step(s) other than the above-described steps (A) to (D). For example, as described above, after step (A) and before step (B), the method may comprise the step of forming the "biocompatible substrate" into a desired shape and the step of grinding and/or polishing the surface of the shape. For example, when a layer(s) is(are) provided between the substrate and the metal film, the step of providing the layer(s) may be provided after step (A) and before step (B).

Hereinafter, the present invention is specifically described with reference to, but is not limited only to, examples.

Examples

<Substrate Material>

As a substrate material assumed to be an implant, smooth plate zirconia A-F and A-F2 cut into $10 \times 10$ mm with a thickness of 3 mm and subjected to general grinding processing on one plane surface were used. A rough plate zirconia A-R was used, one surface of which was roughened by forming micro lattice-like grooves on the surface. Furthermore, a glass substrate B having a dimension of 26 mm $\times$ 76 mm with a thickness of 1.2 mm was also used to examine the details of the characteristics of the formed film.

The surface roughness of each sample was measured in accordance with JIS B0601:2013, with a reference length of 0.8 mm, an evaluation length of 4.0 mm, and cutoff values Ac and As of 0.8 mm and 0.25 um, respectively. In addition, in order to equalize the influence of the grinding direction, the surface roughness was measured in two directions parallel and perpendicular to the grinding direction and averaged. Table 1 summarizes the surface roughness values for each sample.

Table 1. Surface roughness of each substrate material

|  | Ra (um) | Rz ($\mu$m) |
|---|---|---|
| Smooth plate zirconia (A-F) | 0.20 | 1.29 |
| Smooth plate zirconia (A-F2) | 0.35 | 1.91 |
| Rough plate zirconia (A-R) | 10.34 | 31.94 |

<Coating film>

A magnetron sputtering device was used as the sputtering device.

As a sputtering target, pure magnesium, and three kinds of ingots manufactured by dissolving pure magnesium and pure calcium at a predetermined ratio were machined into a disk shape having a diameter of about 120 mm and used. The four kinds of ingots were those in which the amount of calcium was 0% (Mg), 10% (Mg10%Ca), 20% (Mg20%Ca), and 30% (Mg30%Ca), and the remaining amount was magnesium. The % of the calcium amount is a ratio of the calcium weight when the total of the calcium weight and the magnesium weight is 100 % by weight and is represented by wt% as below.

Calcium wt% = Calcium Weight / (Magnesium Weight + Calcium Weight) $\times$ 100

Furthermore, targets with a calcium amount of 40% by weight (Mg40%Ca) or 50% by weight (Mg50%Ca) were tried to make in a manner similar to the target with Mg10%Ca and the like, by using pure magnesium and pure calcium. However, the targets could not be used since cracks occurred during machining and since they were partially damaged.

The substrate was disposed on the stage of the sputtering device so as to face the sputtering target.

In the film formation process, first, the pressure was reduced to a predetermined value, the harmful gas in the chamber was removed, and then an argon gas was sealed.

By appropriately adjusting the voltage and the substrate bias required for discharge, the surfaces of the sputtering target and the substrate were subjected to ion cleaning to remove oxides and harmful compound layers on the surfaces, so that impurities that reduce adhesiveness at the interface between the substrate and the film were removed, and an active surface advantageous for bone formation was formed.

The temperature of the substrate was kept at room temperature, the pressure of argon was 1 to 10 mTorr, the sputtering voltage and the bias of the substrate were adjusted, substrates A-F, A-R and A-F2 as well as glass substrate B were placed in the same chamber so that the film thickness for substrates A-F and A-R is 10 um and the film thickness for substrate A-F2 is 5 $\mu$m, and thus, the film formation process (deposition) was carried out, to form an antibacterial coating film.

Table 2 shows the thickness of magnesium alone film (Mg film), a film with a calcium amount of 20% by weight (Mg20%Ca film), and a film with a calcium amount of 30% by weight (Mg30%Ca film), each of which was obtained by sputtering by using each target of magnesium alone (Mg), 20% by weight of calcium (Mg20%Ca) and 30% by weight of calcium (Mg30%Ca). The thickness of the obtained film was measured by using a sample sputtered onto glass substrate B treated in the same chamber as each substrate. That is, the thickness of the obtained film was measured by actually measuring a gap distance by a stylus method, the gap distance being between a portion on the substrate where the film was formed and another portion where the film was not formed by performing the above-described masking.

Table 2. Thickness and weight of each film

| Substrate | Film composition | Film thickness (um) |
|---|---|---|
| A-F | Mg | 10.482 |
|  | Mg20%Ca | 10.193 |
|  | Mg30%Ca | 10.144 |
| A-F2 | Mg | 5.282 |
|  | Mg20%Ca | 4.875 |
|  | Mg30%Ca | 4.957 |

<Structure of film>

X-ray diffraction analysis was carried out for each coated film by using an X-ray diffractometer (D8 ADVANCE, manufactured by Bruker Corporation) under the conditions of a detector: a two-dimensional detector, tube lamp: Co, tube lamp voltage: 30 kV, tube lamp current: 40 mA, slit: $\Phi$ 1.0 mm, and collimator: $\Phi$ 1.0 mm.

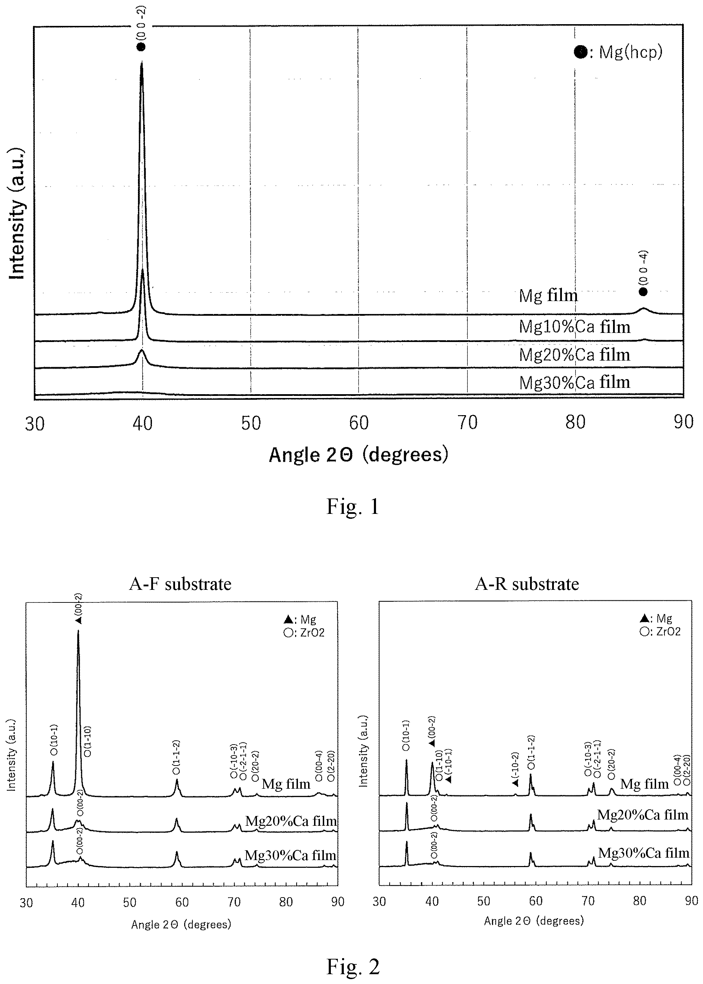

Fig. 1 shows X-ray diffraction profiles of a magnesium alone film (Mg film), a film with a calcium amount of 20% by weight (Mg20%Ca film) and a film with a calcium amount of 30% by weight (Mg30%Ca film), each of which was obtained by sputtering each target of magnesium alone (Mg), 20% by weight of calcium (Mg20%Ca) and 30% by weight of calcium (Mg30%Ca).

Fig. 2 shows X-ray diffraction profiles for an Mg film, an Mg20%Ca film, and an Mg30%Ca film, each of which were obtained by sputtering using smooth plate zirconia A-F and rough plate zirconia A-R.

Figs. 1 and 2 show that a sharp and strong peak derived from crystal is observed around $2\theta$ = 40 degrees derived from the (0 0 -2) plane of magnesium for the Mg film and the Mg10%Ca film. Comparing the peak intensities of the Mg film and the Mg10%Ca film, the peak intensity of the Mg10%Ca film is significantly lower, indicating that the crystallinity is lowered. In addition, in the Mg20%Ca film, a low-intensity peak thought to be derived from microcrystals was observed in the broad halo pattern derived from the amorphous structure, and in the Mg30%Ca film, only the broad halo pattern derived from the amorphous structure was observed. These results show that the Mg film is crystalline, that the Mg10%Ca film is crystalline with low crystallinity, that the Mg20%Ca film has a mixed structure of microcrystals and amorphous, and that the Mg30%Ca film is non-crystalline. In general, when the amount of calcium added to magnesium exceeds 0.8% by weight, calcium does not form a solid solution in magnesium and $Mg_2Ca$ precipitates. However, the presence of intermetallic compounds such as $Mg_2Ca$ was not observed in any of the films.

In comparison of smooth plate zirconia A-F with rough plate zirconia A-R, peak intensities derived from (0 0 -2) plane of magnesium for the Mg films were greatly different from each other, and the peak intensity for smooth plate zirconia A-F is significantly lower than that for rough plate zirconia A-R.

Further, for rough plate zirconia A-R, peaks derived from (-1 0 -1) and (-1 0 -2) planes were observed, which were not observed for smooth plate zirconia A-F. This is because the (0 0 -2) plane of magnesium grows in the direction perpendicular to the substrate surface to form a film by sputtering, and therefore only peaks from the (0 0 -2) plane are observed in smooth plate zirconia A-F. However, the surface roughness of the rough plate zirconia A-R is rough, and the surface itself is oriented in various directions. In the case of the Mg20%Ca film and the Mg30%Ca film, almost no difference was observed in the X-ray diffraction from the films between the smooth plate zirconia A-F and the rough plate zirconia A-R.

For example, in dental implants, the surface is often roughened in order to improve bonding with the bone. As can be seen from Fig. 2, the film can be formed regardless of the surface roughness of the substrate. Therefore, the present antibacterial coating can be similarly applied to roughened dental implants and smooth ones to improve bonding with bone, and can exhibit similar effects.

<PH change accompanying dissolution of film>

A dissolution rate test in simulated body fluid was performed for the samples, each of which was smooth plate zirconia A-F2 coated with a Mg film, a Mg20%Ca film, or a Mg30%Ca film by sputtering. For the samples, the sputtered film was attached to the top and side surfaces of the sample, but not to the bottom surface.

Hank's balanced salt solution (HBSS(-) solution (phenol red free), Fuji Film Wako Pure Chemical) containing no calcium or magnesium was used as the simulated body fluid. The volume of the simulated body fluid was 40 ml. Therefore, assuming that the apparent surface area ignoring the influence of surface roughness is 220 $mm^2$, the simulated body fluid volume per unit area of the film in contact with the solution was 0.182 $ml/mm^2$.

Dissolution rates were evaluated from pH measurements using the glass electrode method. A desktop pH tester (HORIBA, F-74) and a GRT composite electrode (9615S-10D, HORIBA) were used for pH measurement. The dissolution rate test was performed by immersing the sample in HBSS (-) solution kept at 37° in an air bath type constant temperature device, and the pH was measured after 1 hour, 3 hours, 6 hours, 12 hours and 24 hours.

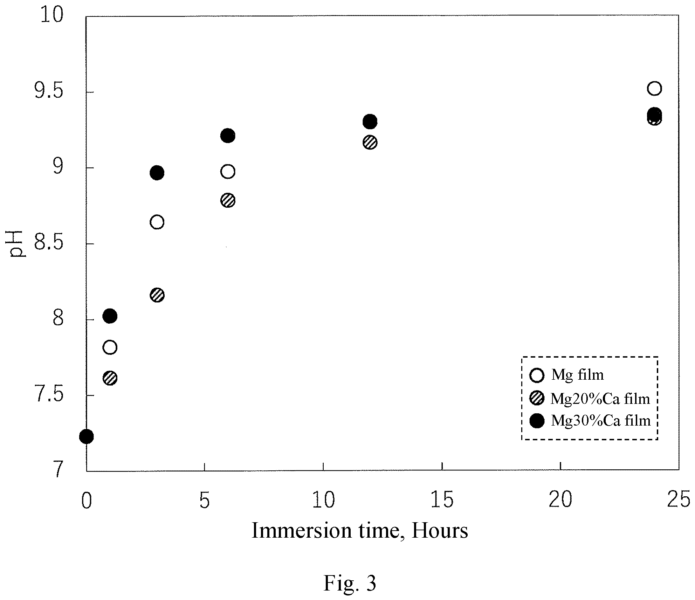

Fig. 3 shows the results of pH measurement as a dissolution rate evaluation in HBSS (-) immersion for smooth plate zirconia A-F2s, each having a Mg film, a Mg20%Ca film and a Mg30%Ca film.

Further, the calculated values of the amount of magnesium and calcium components per unit volume $mol/cm^3$ in the Mg film, the Mg20%Ca film and the Mg30%Ca film when the film thickness is 5.28 um and the total cross-sectional area is 220 $mm^2$ are shown in Table 3.

Table 3. Calculated values of magnesium and calcium existed in the films

| Kinds of film | Mg film | Mg20%Ca film | Mg30%Ca film |
|---|---|---|---|
| Amount of Mg (mol/cm³) | 0.0716 | 0.0559 | 0.0483 |
| Amount of Ca (mol/cm³) | 0 | 0.0084 | 0.0126 |

Fig. 2 and Table 3 shows the following.

For example, E. coli grows optimally at a pH of about 7.0 to 7.5 and cannot live in an alkaline environment with a pH of 9 or higher. Fig. 2 and Table 3 shows that when the films were exposed to the biological environment, a large amount of Mg ions and Ca ions were dissolved, and the pH was greatly inclined to the alkaline side. Also, when a large amount of divalent cations such as Mg ions and Ca ions were present, they themselves may exhibit the antibacterial property. Therefore, the formation of the film on the surface of the implant can exhibit an antibacterial property in the process of dissolution of the film. Further, existence of the dissolved Mg ions and Ca ions around the implant for a long period of time can exhibit long-term antibacterial property.

<Antibacterial property of film>

With reference to the JIS Z2801 film method, the antibacterial properties of samples, each of which was obtained by attaching a Mg film, a Mg20%Ca film and a Mg30%Ca film to smooth plate zirconia A-F, were evaluated.

The bacterial species used in the test was Staphylococcus aureus, and a test bacterial solution with a viable count of $6.8 \times 10^5$ cells/mL was used with an inoculum of 0.016 mL, and the bacterial solution was covered with a PE film of $8 \times 8$ cm², and cultured.

A non-antibacterial PE film and uncoated zirconia were used as comparison materials, and the antibacterial properties of coated zirconia were investigated.

First, the average of viable bacteria count was $1.7 \times 10^4$ cells/cm² when the viable bacteria count immediately after inoculation onto the PE film was evaluated. Subsequently, the average was $2.5 \times 10^4$ cells/cm² when the viable bacteria count at 24 hours after inoculation onto uncoated zirconia was evaluated. Based on these preliminary experiments, the antibacterial activity value was evaluated from the formula (1).

$$R = (U_t - U_0) - (A_t - U_0) = U_t - A_t \qquad (1)$$

Here, R: an antibacterial activity value, $U_0$: an average logarithm value of viable bacteria count immediately after inoculation onto PE film, Ut: an average logarithm value of the viable bacteria count at 24 hours after inoculation onto non-coated zirconia, At: an average logarithm value of the viable bacteria count at 24 hours after inoculation onto zirconia with each coating. In addition, in the measurement of the viable bacteria count, when the number of colonies was 1 or less, it was regarded as 1, and the viable bacteria count per unit area (cm²) of the PE film was calculated.

Table 4 shows the antibacterial evaluation test results for zirconia A-F2s each of which was coated with Mg film, Mg20%Ca film, or Mg30%Ca film.

Table 4. Antibacterial test results for each coating film

| | | |
|---|---|---|
| Immediate after inoculation onto PE film | Viable bacteria count (cells/cm²) | 17000 |
| | Average logarithm value of viable bacteria count, $U_0$ | 4.2 |
| At 24 hours after inoculation onto non-coated zirconia | Viable bacteria count (cells/cm²) | 24500 |
| | Average logarithm | 4.3 |
| | value of viable bacteria count, Ut | |
| At 24 hours after inoculation onto zirconia coated with Mg film | Viable bacteria count (cells/cm²) | 16 |
| | Average logarithm value of viable bacteria count, $A_t$ | 1.2 |
| | Antibacterial activity value R | 3.1 |

(continued)

| At 24 hours after inoculation onto zirconia coated with Mg20%Ca film | Viable bacteria count (cells/cm$^2$) | 16 |
|---|---|---|
| | Average logarithm value of viable bacteria count, $A_t$ | 1.2 |
| | Antibacterial activity value, R | 3.1 |
| At 24 hours after inoculation onto zirconia coated with Mg30%Ca film | Viable bacteria count (cells/cm$^2$) | 16 |
| | Average logarithm value of viable bacteria count, $A_t$ | 1.2 |
| | Antibacterial activity value, R | 3.1 |

Table 4 shows the following.

For example, if an antibacterial property can be imparted to the surface of a dental implant, it is possible to prevent the occurrence of peri-implantitis by killing bacteria that adhere to the surface during or after surgery. Table 4 shows that bacteria were alive on uncoated zirconia, while on zirconia coated with Mg film, Mg20%Ca film, or Mg30%Ca film, bacteria were killed in all cases. Therefore, the formation of the coating film on the implant surface can impart high antibacterial property thereto.

**Claims**

1.  A biocompatible film comprising magnesium and optionally calcium, wherein calcium has 0 to 40 % by weight, where a total weight of magnesium and calcium is 100 % by weight, and the film has an antibacterial property having an antibacterial activity value of 2.0 or more.

2.  The biocompatible film according to claim 1, wherein the film has the antibacterial property in which an average of a viable bacteria count of Staphylococcus aureus at 24 hours after inoculating 0.16 $\mu$l/mm$^2$ of a test bacterial solution having a viable bacteria count of Staphylococcus aureus of $2.5 \times 10^5$ to $10 \times 10^5$ cells/ml onto the film is 75 % or less of the viable bacteria count at the time of inoculation.

3.  A biocompatible film comprising magnesium and optionally calcium, wherein calcium has 0 to 40 % by weight, where a total weight of magnesium and calcium is 100 % by weight, and the film has an antibacterial property in which an average of a viable bacteria count of Staphylococcus aureus at 24 hours after inoculating 0.16 $\mu$l/mm$^2$ of a test bacterial solution having a viable bacteria count of Staphylococcus aureus of $2.5 \times 10^5$ to $10 \times 10^5$ cells/ml onto the film is 75 % or less of the viable bacteria count at the time of inoculation.

4.  The biocompatible film according to claim 1 or 3, having a dissolution property such that the biocompatible film dissolves in a Hank's balanced salt solution.

5.  The biocompatible film according to claim 1 or 3, wherein the biocompatible film is free from Mg$_2$Ca.

6.  The biocompatible film according to claim 1 or 3, wherein the biocompatible film has an amorphous portion.

7.  A biocompatible material comprising the biocompatible film according to claim 1 or 3; and a biocompatible substrate.

8.  The biocompatible material according to claim 7, wherein the biocompatible substrate is at least one selected from the group consisting of pure titanium, a cobalt-chromium alloy, stainless steel, titanium alloys, zirconia, alumina, calcium phosphate and magnesia.

9.  The biocompatible material according to claim 7, wherein a surface roughness Ra of the biocompatible substrate is 50 um or less.

10. The biocompatible material according to claim 7, wherein a shape of the biocompatible material is one selected from the group consisting of a cylindrical shape, a truncated cone shape, a conical shape, a shape having a screw-shaped threaded portion in a part of the shape, a rectangular parallelepiped and a cube, a block shape having a partially inclined surface, and a wedge shape.

11. The biocompatible material according to claim 7, wherein the biocompatible material is one selected from the group consisting of an artificial bone material, an intraosseous fixture material, a dental implant material, an orthodontic anchor screw material, a medullary nail material, and an interbody fixation material.

Fig. 1

Fig. 2

Fig. 3

## INTERNATIONAL SEARCH REPORT

| International application No. |
|---|
| **PCT/JP2022/032507** |

| A. | CLASSIFICATION OF SUBJECT MATTER |
|---|---|

*A61L 27/02*(2006.01)i; *A61C 8/00*(2006.01)i; *A61L 27/04*(2006.01)i; *A61L 27/06*(2006.01)i; *A61L 27/42*(2006.01)i; *A61L 27/54*(2006.01)i

FI:    A61L27/02; A61L27/06; A61L27/04; A61L27/42; A61L27/54; A61C8/00 Z

According to International Patent Classification (IPC) or to both national classification and IPC

| B. | FIELDS SEARCHED |
|---|---|

Minimum documentation searched (classification system followed by classification symbols)

A61L27/02; A61C8/00; A61L27/04; A61L27/06; A61L27/42; A61L27/54

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | ZAATREH, Sarah et al. Fast corroding, thin magnesium coating displays antibacterial effects and low cytotoxicity. Biofouling. 2017, vol. 33(4), pages 294-305 | 1-11 |
| | page 294, column of abstract, page 302, left column, lines 1-4, page 303, column of conclusion, tables 1-2, fig. 1-6, page 298, left column, line 1 to right column, line 5 | |
| Y | | 1-11 |
| Y | ROBINSON, Duane A. et al. In vitro antibacterial properties of magnesium metal against Escherichia coli, Pseudomonas aeruginosa and Staphylococcus aureus. Acta Biomaterialia. 2010, vol. 6, pages 1869-1877 | 1-11 |
| | colmun of abstract, fig. 1-5 | |
| Y | LI, Yang et al. Antibacterial Properties of Magnesium In Vitro and in an In Vivo Model of Implant-Associated Methicillin-Resistant Staphylococcus aureus Infection. Antimicrobial Agents and Chemotherapy. 2014, vol. 58(12), pages 7586-7591 | 1-11 |
| | page 7586, left column, lines 21-31, fig. 1-3 | |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 November 2022** | **22 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2022/032507**

### C. DOCUMENTS CONSIDERED TO BE RELEVANT

| Category* | Citation of document, with indication, where appropriate, of the relevant passages | Relevant to claim No. |
|---|---|---|
| X | JP 2012-196461 A (U & I CORP.) 18 October 2012 (2012-10-18)<br>    examples 1-2, 4-5, paragraph [0047] | 1-11 |
| Y | | 1-11 |
| A | JP 8-182755 A (ISHIKAWAJIMA HARIMA HEAVY IND. CO., LTD.) 16 July 1996 (1996-07-16)<br>    whole document | 1-11 |
| A | JP 2015-136469 A (IBARAKI UNIV.) 30 July 2015 (2015-07-30)<br>    whole document | 1-11 |
| A | SCHLUTER, K. et al. Polycrystalline and amorphous MgZnCa thin films. Corrosion Science. 2012, vol. 63, pages 234-238<br>    whole document | 1-11 |
| P, X | JP 2022-102829 A (MARUEMU WORKS CO., LTD.) 07 July 2022 (2022-07-07)<br>    whole document | 1-11 |
| P, X | JP 2022-074692 A (MARUEMU WORKS CO., LTD.) 18 May 2022 (2022-05-18)<br>    whole document | 1-11 |
| P, X | WO 2022/097670 A1 (MARUEMU WORKS CO., LTD.) 12 May 2022 (2022-05-12)<br>    whole document | 1-11 |

Form PCT/ISA/210 (second sheet) (January 2015)

Form PCT/ISA/210 (patent family annex) (January 2015)

| INTERNATIONAL SEARCH REPORT<br>Information on patent family members | | International application No.<br>**PCT/JP2022/032507** | |
|---|---|---|---|
| Patent document<br>cited in search report | Publication date<br>(day/month/year) | Patent family member(s) | Publication date<br>(day/month/year) |
| JP 2012-196461 A | 18 October 2012 | US 2010/0075162 A1<br>examples 1-2, 4-5, paragraph [0066]<br>EP 2063816 A1<br>KR 10-2008-0027202 A<br>CN 101516292 A | |
| JP 8-182755 A | 16 July 1996 | (Family: none) | |
| JP 2015-136469 A | 30 July 2015 | (Family: none) | |
| JP 2022-102829 A | 07 July 2022 | (Family: none) | |
| JP 2022-074692 A | 18 May 2022 | (Family: none) | |
| WO 2022/097670 A1 | 12 May 2022 | (Family: none) | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6130301 B
- JP 2008161424 A

- JP 2021007741 A